# EUROPEAN PATENT APPLICATION

(11) **EP 4 792 822 A1**
(43) Date of publication of application: **19.08.2026**
(21) Application number: 24876540.6
(22) Date of filing: 09.10.2024
(51) Int. Cl.: A61K 38/07, C07K 5/107, A61P 1/08

(54) **USE OF PEPTIDE AMIDE COMPOUND IN PREPARATION OF DRUG FOR PREVENTING NAUSEA AND VOMITING**

(30) Priority: 10.10.2023 CN 202311305170; 06.05.2024 CN 202410544545; 29.07.2024 CN 202411022811
(71) Applicant: Haisco Pharmaceutical Group Co., Ltd., Shannan, Xizang 856099 (CN)
(72) Inventor: CAO, Yong, Chengdu, Sichuan 611130 (CN); ZHAO, Wanyun, Chengdu, Sichuan 611130 (CN); LI, Yanjun, Chengdu, Sichuan 611130 (CN); CHEN, Wei, Chengdu, Sichuan 611130 (CN)
(74) Representative: AWA Denmark A/S
(86) International application number: PCT/CN2024/123615
(87) International publication number: WO 2025/077727

(57) **Abstract**

A use of a compound of formula (I) or a stereoisomer, hydrate, metabolite, solvate, pharmaceutically acceptable salt and eutectic thereof or a composition thereof in the preparation of a drug for preventing postoperation nausea and vomiting or chemotherapy-induced nausea and vomiting.

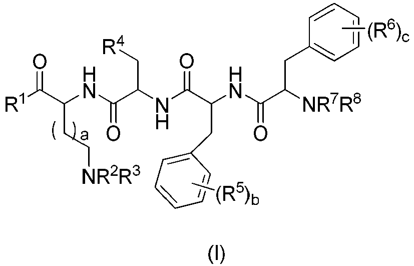

## Description

### Technical Field

The present invention relates to the use of a compound of formula (I) or a stereoisomer, hydrate, metabolite, solvate, pharmaceutically acceptable salt or eutectic thereof, and a composition thereof in the preparation of a drug for preventing postoperation nausea and vomiting or chemotherapy-induced nausea and vomiting.

### Background Art

Postoperation nausea and vomiting (PONV) is a gastrointestinal dysfunction that occurs after surgery, occurring mainly within 6 h or 24 h after surgery, with symptoms persisting in a small number of patients for 3 to 5 days. The incidence of PONV is 30% in general surgical patients and as high as 80% in high-risk patients. PONV can cause patients to experience varying degrees of suffering, including water and electrolyte imbalance, wound dehiscence, incisional hernia formation, aspiration, and aspiration pneumonia, thereby reducing patient satisfaction, prolonging hospital stays, and increasing medical costs. It is evident that PONV is an important factor affecting postoperative recovery in patients.

The mechanism of PONV is complex, involving central and peripheral receptors and multiple neural transmission pathways. The central sites involved in the vomiting reflex include the vomiting centre and the chemoreceptor trigger zone (CTZ). When receptors in the digestive system, such as the tongue base, pharynx, stomach, intestines, and common bile duct, or in the cerebral cortex and vestibular organs, are stimulated, afferent signals are transmitted to the vomiting centre via the vagus nerve, sympathetic nerves, glossopharyngeal nerve, etc. In addition, the CTZ is rich in multiple receptors, such as dopamine receptors, 5-HT3 receptors, histamine receptors, opioid receptors, and cholinergic receptors, which can directly sense various metabolites, drugs, toxins, etc., in the blood and cerebrospinal fluid, and transmit the signals to the vomiting centre. Subsequently, impulse signals from the vomiting centre are transmitted via the vagus nerve, sympathetic nerves, phrenic nerve, and spinal nerves to the stomach, small intestine, diaphragm, and abdominal wall muscles, thereby triggering vomiting.

Chemotherapy-induced nausea and vomiting (CINV) is one of the complications frequently encountered in cancer patients during chemotherapy. Over 70% of cancer patients receiving antitumour therapy experience varying degrees of nausea and vomiting. Severe nausea and vomiting may lead to conditions such as dehydration, electrolyte imbalance, and nutritional deficiency, hindering the normal course of antitumour therapy. According to the time of onset, CINV is usually classified into five types: acute, delayed, breakthrough, refractory, and anticipatory. Acute nausea and vomiting generally occur within minutes to hours after drug administration and peak at 5 to 6 h after administration, but mostly resolve within 24 h. Delayed nausea and vomiting often occur 24 h after drug administration, are commonly observed with chemotherapy drugs including cisplatin, carboplatin, and cyclophosphamide, and can last for several days, generally 2 to 5 days.

It is currently believed that CINV is caused by the stimulation of cerebral cortical pathways. For example, chemotherapeutic agents stimulate chromaffin cells in the gastrointestinal tract to release neurotransmitters. These neurotransmitters bind to corresponding receptors to generate nerve impulses, which are transmitted via the vagus and sympathetic nerves to directly stimulate the chemoreceptor trigger zone (CTZ). This results in increased salivation, accelerated respiration, and contractions of the pharynx, gastrointestinal tract, and abdominal muscles, ultimately leading to vomiting. Currently, 5-hydroxytryptamine (5-HT) is considered the main neurotransmitter mediating acute CINV, but its involvement in the occurrence of delayed CINV remains unclear. In addition to 5-HT, neurotransmitters involved in vomiting mainly include dopamine, histamine, substance P, etc. The neurokinin-1 (NK-1) receptor for substance P can regulate the vomiting response via both central and peripheral pathways, with the former being predominant. In addition to the above mechanisms of chemotherapy-induced vomiting, chemotherapeutic agents and metabolites thereof can also directly stimulate the medullary chemoreceptor trigger zone, which in turn transmits signals to the central nervous system to induce vomiting. Meanwhile, sensory and psychological factors in patients can directly stimulate cerebral cortical pathways to induce vomiting.

Currently, the prevention and treatment measures for PONV and CINV are mainly based on drug therapy. Drugs act on the cerebral cortex, chemoreceptor trigger zone, vomiting centre, and visceral afferent nerves to block one or more receptors such as 5-HT3, dopamine, histamine, cholinergic and neurokinin receptors, thereby achieving an antiemetic effect. Commonly used drugs include 5-HT3 receptor antagonists (ondansetron, dolasetron, etc.), neurokinin-1 (NK-1) receptor antagonists (aprepitant, rolapitant, etc.), corticosteroids (dexamethasone, etc.), antidopaminergic agents (haloperidol, metoclopramide, etc.), anticholinergics (scopolamine transdermal patch), antihistamines (dimenhydrinate, promethazine, etc.), etc.

For surgical patients, postoperative recovery is affected by multiple factors such as pain, vomiting, and infection. To facilitate early patient recovery, clinical practice often requires targeted use of multiple drugs, which may, however, carry a higher risk of adverse events associated with the use of these drugs. For chemotherapy patients, severe and persistent nausea and vomiting may have a serious impact on the physical and mental health of patients due to the need for periodic chemotherapy treatment.

Although drugs are available to prevent postoperation nausea and vomiting and chemotherapy-induced nausea and vomiting, the use thereof is limited due to unsatisfactory efficacy or side effects. Therefore, there is a need for new drugs with improved efficacy and fewer side effects.

### Summary of the Invention

The present invention provides the use of a compound of formula (I) or a stereoisomer, hydrate, metabolite, solvate, pharmaceutically acceptable salt or eutectic thereof, and a composition thereof in the preparation of a drug for preventing postoperation nausea and vomiting or chemotherapy-induced nausea and vomiting: 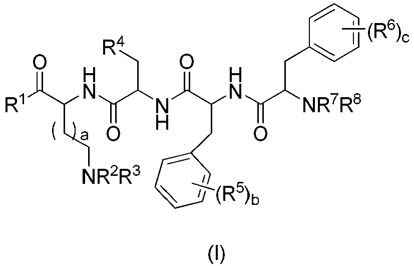 wherein
R₁ is selected from 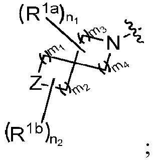
m1, m2, m3 and m4 are each independently selected from 0, 1, 2, 3 or 4; m1 and m2 are not both 0; m3 and m4 are not both 0;
n1 and n2 are each independently selected from 0, 1, 2, 3 or 4;
Z is selected from CR^{z1}R^{z2} or NR^{z3};
R^{z1} and R^{z2} are each independently selected from H, F, Cl, Br, I, OH, CF₃, nitro, C₁₋₆ alkyl, C₁₋₆ alkoxy, C₂₋₆ alkenyl, C₂₋₆ alkynyl, -C(=O)-C₁₋₆ alkyl, -(CH₂)_{q}-C(=O)O-C₁₋₆ alkyl, -(CH₂)_{q}-NR^{le}R^{lf}, -(CH₂)_{q}-COOH, -(CH₂)_{q}-CONH₂, C₃₋₈ carbocyclyl or 3- to 8-membered heterocyclyl, wherein the alkyl, the alkoxy, the alkenyl, the alkynyl, the carbocyclyl or the heterocyclyl is optionally further substituted with 0-5 substituents selected from F, Cl, Br, I, OH, CF₃, =O, carboxyl, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, the heterocyclyl contains 1 to 3 heteroatoms optionally selected from N, O or S, and when the heteroatom is selected from S, the heterocyclyl is optionally further S, S=O or S(=O)₂;
R^{le} and R^{lf} are each independently selected from H, C₁₋₆ alkyl, C(=O)O-C₁₋₆ alkyl, -C(=O)O-(CH₂)_{q}-C₃₋₈ carbocyclyl or -C(=O)O-(CH₂)_{q}-3- to 8-membered heterocyclyl, wherein the alkyl, the carbocyclyl or the heterocyclyl is optionally further substituted with 0-5 substituents selected from F, Cl, Br, I, OH, CF₃, cyano, nitro, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms selected from N, O or S;
or R^{z1} and R^{z2}, together with the carbon atom to which they are attached, form a 3- to 10-membered nitrogen-containing heterocyclic ring, wherein the ring is optionally further substituted with a substituent selected from F, Cl, Br, I, OH, CF₃, cyano, nitro, =O, C₁₋₆ alkyl, C₁₋₆ alkoxy, C₂₋₆ alkenyl, C₂₋₆ alkynyl, C_{.} carbocyclyl or 3- to 8-membered heterocyclyl;
R^{la} and R^{lb} are each independently selected from F, CF₃, NH₂, C₁₋₆ alkyl, C₂₋₆ alkenyl, C₂₋₆ alkynyl or 3- to 8-membered heterocyclyl, wherein the alkyl, the alkenyl, the alkynyl or the heterocyclyl is optionally further substituted with 0-5 substituents selected from F, Cl, Br, I, OH, CF₃, nitro, cyano, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
R^{z3} is independently selected from H, -C(=O)-C₁₋₆ alkyl, -C(=O)O-C₁₋₆ alkyl, -C(=O)-C₃₋₈ carbocyclyl, -C(=O)O-C₃₋₈ carbocyclyl, -C(=O)O-(3- to 8-membered heterocyclyl), -S(=O)ₚ-C₁₋₆ alkyl, -S(=O)ₚ-C₃₋₈ carbocyclyl, -S(=O)ₚ-(3- to 8-membered heterocyclyl), -C(=O)NR^{1g}R^{lh}, -S(=O)p-NR^{li}R^{lj} or 3- to 8-membered heterocyclyl, wherein the alkyl, the carbocyclyl or the heterocyclyl is optionally further substituted with 0-5 substituents selected from F, Cl, Br, I, OH, CF₃, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
R^{lg}, R^{lh}, R^{li'} and R^{lj} are each independently selected from H or C₁₋₆ alkyl;
or R^{lg} and R^{lh}, together with the nitrogen atoms to which they are attached, form a 3- to 10-membered heterocyclic ring, wherein the ring is optionally further substituted with a substituent selected from F, Cl, Br, I, OH, CF₃, cyano, nitro, amino, C₁₋₆ alkyl, C₁₋₆ alkoxy, C₂₋₆ alkenyl, C₂₋₆ alkynyl or -S(=O)ₚ-C₁₋₆ alkyl, and the heterocyclyl contains 1 to 3 heteroatoms selected from N, O or S;
q is selected from 0, 1, 2, 3 or 4;
p is selected from 0, 1 or 2;
a is selected from 0, 1, 2 or 3;
R⁴ is independently selected from H, C₁₋₆alkyl, C₂₋₆alkenyl, C₂₋₆alkynyl or - (CH₂)_{q}-C₃₋₈ carbocyclyl, wherein the alkyl, the alkenyl, the alkynyl or the carbocyclyl is optionally further substituted with 0-5 substituents selected from F, Cl, Br, I, OH, CN, CF₃, NO₂, C₁₋₆alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms selected from N, O or S;
R², R³, R⁷ and R⁸ are each independently selected from H, C₁₋₆ alkyl, - C(=O)O-C₁₋₄ alkyl, -C(=O)O-(CH₂)_{q}-C₃₋₈ carbocyclyl, -C(=O)O-(CH₂)_{q}-3- to 8-membered heterocyclyl or 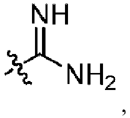 wherein the alkyl, the carbocyclyl or the heterocyclyl is optionally further substituted with 0-5 substituents selected from F, Cl, Br, I, OH, CF₃, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
b is selected from 0, 1, 2, 3, 4 or 5;
c is selected from 0, 1, 2, 3, 4 or 5;
R⁵ and R⁶ are each independently selected from F, Cl, Br, I, OH, CN, CF₃, cyano, nitro, C₁₋₄ alkyl, -OR^{5a}, -C(O)OR^{5b}, -SR^{5c}, -S(O)R^{5d}, -S(O)₂R^{5e} or -NR^{5f}R^{5g};
R^{5a}, R^{5b}, R^{5c}, R^{5d}, R^{5e}, R^{5f} and R^{5g} are each independently selected from H or C₁₋₄ alkyl;
or R^{5f} and R^{5g}, together with the nitrogen atom to which they are attached, form a 5- to 6-membered heterocyclic ring, wherein the heterocyclyl contains 1 to 3 heteroatoms optionally selected from N, O or S.

Preferably, the compound is selected from formula (II): 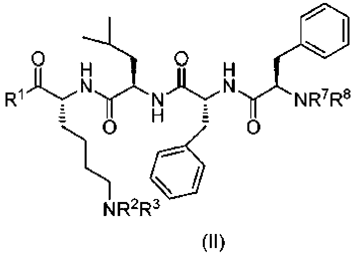 wherein
R₁ is selected from 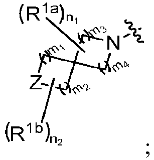
m1, m2, m3 and m4 are each independently selected from 0, 1 or 2; m1 and m2 are not both 0; m3 and m4 are not both 0;
n1 and n2 are each independently selected from 0 or 2;
Z is selected from CR^{zl}R^{z2} or NR^{z3};
R^{1a}, R^{1b} are independently selected from F or NH₂;
R^{z1} and R^{z2} are each independently selected from H, carboxyl, 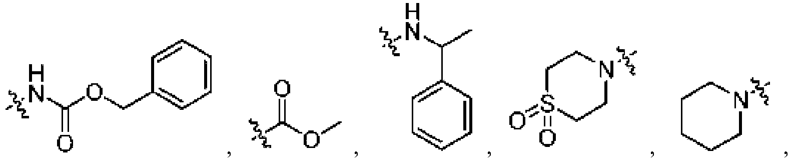 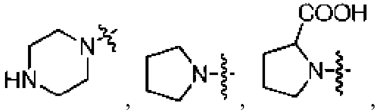 amino, -CH₂NH₂ or 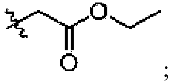
or R^{z1} and R^{z2} can, together with the carbon atom to which they are attached, form a lactam 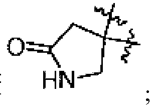
R^{z3} is independently selected from H, -C(=O)-C₁₋₄ alkyl, -C(=O)O-C₁₋₄ alkyl, -C(=O)-C₃₋₆ carbocyclyl, -C(=O)O-C₃₋₆ carbocyclyl, -S(=O)ₚ-C₁₋₄ alkyl, -S(=O)ₚ-C₃₋₆ carbocyclyl, -C(=O)NR^{1g}R^{lh}, -S(=O)ₚ-NR^{li}R^{lj} or 3- to 6-membered heterocyclyl, wherein the alkyl, the carbocyclyl or the heterocyclyl is optionally further substituted with 0-5 substituents selected from F, Cl, Br, I, OH, CF₃, nitro, cyano, amino, methyl, ethyl, methoxy, ethoxy, cyclopropyl or phenyl, and the heterocyclyl contains 1 to 3 heteroatoms optionally selected from N, O or S;
R^{lg}, R^{lh}, R^{li'} and R^{lj} are each independently selected from H or C₁₋₄ alkyl;
or R^{lg} and R^{lh}, together with the nitrogen atom to which they are attached, form a 4- to 6-membered heterocyclic ring, wherein the ring is optionally further substituted with a substituent selected from F, CF₃, methyl, methoxy or -S(=O)ₚ-C₁₋₄ alkyl, and the heterocyclyl contains 1 to 3 heteroatoms selected from N, O or S;
p is selected from 2;
R², R³, R⁷ and R⁸ are each independently selected from H, methyl or C(=O)O-tert-butyl.

Preferably, the compound of formula (II) is selected from one of the following structures:

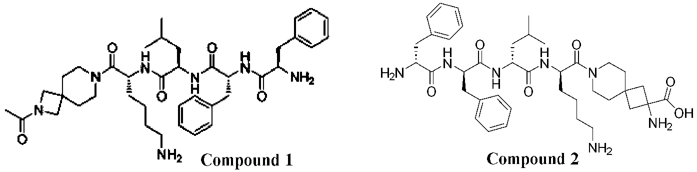

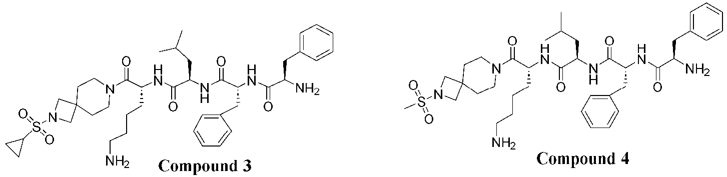

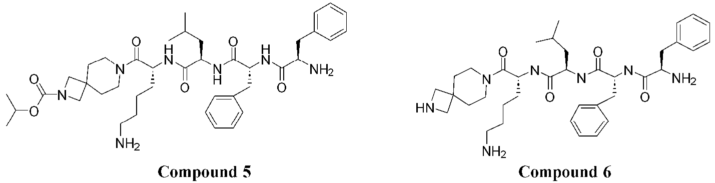

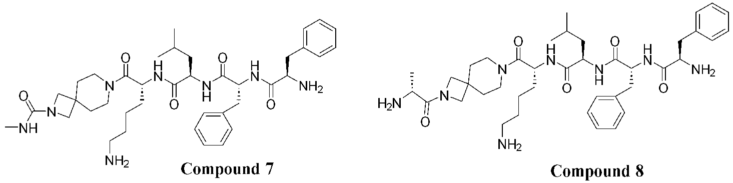

Preferably, the compound of formula (II) is selected from:

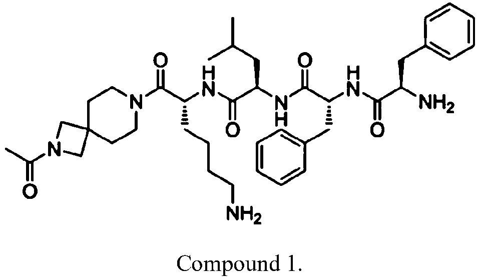

In one embodiment of the present invention, an effective dose of the compound is selected from 1 ng-30 mg, 0.01 µg-10 mg, 0.1 µg-1 mg, 1 µg-1000 µg, 10 µg-800 µg, 10 µg-600 µg, 10 µg-400 µg and 10 µg-200 µg, and effective doses of the hydrate, metabolite, solvate, pharmaceutically acceptable salt or eutectic of the compound of the formula, and the composition thereof are converted based on the effective dose of the compound.

In one embodiment of the present invention, the administration form is one or more of single administration, multiple administrations, continuous administration, and target-controlled infusion.

In one embodiment of the present invention, the administration is performed via a route selected from: intravenous injection, intraarterial injection, intramuscular injection, transdermal absorption, buccal absorption, parenteral intraperitoneal administration, rectal administration, transbuccal administration, intranasal administration, inhalation, topical delivery, subcutaneous administration, intraadipose administration, intraarticular administration, intraperitoneal administration, or intrathecal administration

In one embodiment of the present invention, the administration is performed via intravenous injection or intranasal administration.

In one embodiment of the present invention, an effective dose for intravenous injection is 40 µg-400 µg or 60 µg-180 µg.

In some embodiments of the present invention, the compound of formula (I) or the stereoisomer, hydrate, metabolite, solvate, pharmaceutically acceptable salt or eutectic thereof, and the composition thereof are administered before the end of surgery.

In some embodiments of the present invention, the use further comprises a second drug, wherein the second drug is selected from one or more of antiemetic agents or analgesic agents.

In some embodiments of the present invention, the antiemetic agent is selected from tropisetron, ondansetron, granisetron, dolasetron, azasetron, ramosetron, palonosetron, aprepitant, fosaprepitant, rolapitant, casopitant, vetipitant, dimenhydrinate, promethazine, scopolamine, gabapentin, pregabalin, midazolam and ephedrine;
in some embodiments of the present invention, the analgesic agent is selected from morphine, fentanyl, sufentanil, oxycodone, hydromorphone, pethidine, butorphanol, nalbuphine, dezocine, pentazocine, buprenorphine, flurbiprofen axetil, ketorolac, lornoxicam, diclofenac, parecoxib, celecoxib, paracetamol, tramadol, dexmedetomidine, ketamine, esketamine, gabapentin and pregabalin.

Unless otherwise stated, the terms used in the description and the claims have the following meanings.

"Alkyl" refers to a linear or branched monovalent saturated hydrocarbon group, in which the main chain comprises a linear or branched group of 1 to 10 carbon atoms, preferably 1 to 8 carbon atoms, further preferably 1 to 6 carbon atoms, more preferably 1 to 4 carbon atoms, and most preferably 1 to 2 carbon atoms. Examples of alkyl include, but are not limited to methyl, ethyl, n-propyl, isopropyl, n-butyl, isobutyl, sec-butyl, tert-butyl, n-pentyl, 2-pentyl, 3-pentyl, 2-methyl-2-butyl, 3-methyl-2-butyl, n-hexyl, n-heptyl, n-octyl, n-nonyl and n-decyl. The alkyl can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxyalkyl, C₁₋₆ alkoxy, C₂₋₆ alkenyl, C₂₋₆ alkynyl, C₃₋₈ carbocyclyl, 3- to 8-membered heterocyclyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}, wherein R¹⁹ and R^{19a} are each independently selected from H, hydroxyl, amino, carboxyl, C₁₋₈ alkyl, C₁₋₈ alkoxy, C₂₋₈ alkenyl, C₂₋₈ alkynyl, 3- to 10-membered carbocyclyl, 4- to 10-membered heterocyclyl, 3- to 10-membered carbocyclyloxy or 4- to 10-membered heterocyclyloxy, k is selected from 0, 1, 2, 3, 4 or 5, and j is selected from 0, 1 or 2. The alkyl, k, j, R¹⁹ and R^{19a} herein are as defined above.

"Alkylene" refers to linear and branched divalent saturated hydrocarbon groups, including -(CH₂)ᵥ- (v is an integer from 1 to 10), and examples of alkylene include, but are not limited to, methylene, ethylene, propylene, butylene, etc. The alkylene can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxyalkyl, C₁₋₆ alkoxy, C₂₋₆ alkenyl, C₂₋₆ alkynyl, C₃₋₈ carbocyclyl, 3- to 8-membered heterocyclyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)j-R¹⁹, -O-C(=O)-O-R¹⁹ or -NR¹⁹R^{19a}. When the number of substituents in the alkylene is greater than or equal to 2, the substituents may be fused together to form a cyclic structure. The alkylene herein is as defined above.

"Alkoxy" refers to a monovalent group of O-alkyl, wherein the alkyl is as defined herein, and examples of alkoxy include, but are not limited to methoxy, ethoxy, 1-propoxy, 2-propoxy, 1-butoxy, 2-methyl-1-propoxy, 2-butoxy, 2-methyl-2-propoxy, 1-pentyloxy, 2-pentyloxy, 3-pentyloxy, 2-methyl-2-butoxy, 3-methyl-2-butoxy, 3-methyl-1-butoxy, 2-methyl-1-butoxy, etc.

"Alkenyl" refers to linear and branched monovalent unsaturated hydrocarbon groups having at least 1, usually 1, 2 or 3 carbon-carbon double bonds, in which the main chain comprises 2 to 10 carbon atoms, further preferably 2 to 6 carbon atoms, and more preferably 2 to 4 carbon atoms. Examples of alkenyl include, but are not limited to ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc. The alkenyl can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxyalkyl, C₁₋₆ alkoxy, C₂₋₆ alkenyl, C₂₋₆ alkynyl, C₃₋₈ carbocyclyl, 3- to 8-membered heterocyclyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. The alkenyl herein is as defined above.

"Alkynyl" refers to a linear or branched monovalent unsaturated hydrocarbon group having at least 1, usually 1, 2 or 3 carbon-carbon triple bonds, in which the main chain comprises 2 to 10 carbon atoms, further preferably 2 to 6 carbon atoms, and more preferably 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc. The alkynyl can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxyalkyl, C₁₋₆ alkoxy, C₂₋₆ alkenyl, C₂₋₆ alkynyl, C₃₋₈ carbocyclyl, 3- to 8-membered heterocyclyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. The alkynyl herein is as defined above.

"Cycloalkyl" refers to a monovalent saturated carbocyclic hydrocarbon group, usually having from 3 to 10 carbon atoms, and non-limiting examples include cyclopropyl, cyclobutyl, cyclopentyl, cyclohexyl, cycloheptyl, etc. The cycloalkyl can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxyalkyl, C₁₋₆ alkoxy, C₂₋₆ alkenyl, C₂₋₆ alkynyl, C₃₋₈ carbocyclyl, 3- to 8-membered heterocyclyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or -NR¹⁹R^{19a}. The cycloalkyl herein is as defined above.

"Carbocycle" refers to a saturated or unsaturated aromatic ring or non-aromatic ring, and the aromatic ring or non-aromatic ring can be a 3- to 10-membered monocyclic ring, 4- to 12-membered bicyclic ring or 10- to 15-membered tricyclic ring system. Carbocyclyl can be linked to a bridged ring or spiro ring. Non-limiting examples include cyclopropyl, cyclobutyl, cyclopentyl, 1-cyclopentyl-1-enyl, 1-cyclopentyl-2-enyl, 1-cyclopentyl-3-enyl, cyclohexyl, 1-cyclohexyl-2-enyl, 1-cyclohexyl-3-enyl, cyclohexenyl, cyclohexadienyl, cycloheptyl, cyclooctyl, cyclononyl, cyclodecyl, cycloundecyl, cyclododecyl, phenyl or naphthyl. The carbocyclyl can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxyalkyl, C₁₋₆ alkoxy, C₂₋₆ alkenyl, C₂₋₆ alkynyl, C₃₋₈ carbocyclyl, 3- to 8-membered heterocyclyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, -O-C(=O)-O-R¹⁹ or - NR¹⁹R^{19a}. The carbocycle herein is as defined above.

The "heterocycle" refers to a saturated or unsaturated aromatic ring or non-aromatic ring; the aromatic ring or non-aromatic ring can be a 3- to 10-membered monocyclic ring, 4- to 12-membered bicyclic ring or 10- to 15-membered tricyclic ring system, and contains 1 to 4 heteroatoms selected from N, O or S, preferably 3-to 8-membered heterocyclyl; and the optionally substituted N and S in the heterocyclyl can be oxidised into various oxidation states. Heterocyclyl can be linked to a heteroatom or a carbon atom, and can be linked to a bridged ring or a spiro ring. Non-limiting examples include epoxyethyl, epoxypropyl, azacyclopropyl, oxacyclobutyl, azacyclobutyl, thiacyclobutyl, 1,3-dioxolanyl, 1,4-dioxolanyl, 1,3-dioxanyl, azacycloheptyl, oxacycloheptyl, thiacycloheptyl, oxazepinyl, diazepinyl, thiazepinyl, pyridyl, piperidinyl, homopiperidinyl, furyl, thienyl, pyranyl, N-alkylpyrrolyl, pyrimidinyl, pyrazinyl, pyridazinyl, piperazinyl, homopiperazinyl, imidazolyl, piperidinyl, morpholinyl, thiomorpholinyl, oxathianyl, dihydrofuranyl, dihydropyranyl, dithiocyclopentyl, tetrahydrofuranyl, tetrahydrothienyl, tetrahydropyranyl, tetrahydrothiopyranyl, tetrahydropyrrolyl, tetrahydroimidazolyl, tetrahydrothiazolyl, tetrahydropyranyl, benzimidazolyl, benzopyridyl, pyrrolopyridyl, benzodihydrofuryl, 2-pyrrolinyl, 3-pyrrolinyl, dihydroindolyl, 2H-pyranyl, 4H-pyranyl, dioxacyclohexyl, 1,3-dioxoclopentyl, pyrazolinyl, dithianyl, dithiolanyl, dihydrothienyl, pyrazolidinyl, imidazolinyl, imidazolidinyl, 1,2,3,4-tetrahydroisoquinolinyl, 3-azabicyclohexyl, 3-azabicycloheptyl, azabicyclohexyl, 3H-indolylquinazinyl, N-pyridyl urea, 1,1-dioxothiomorpholinyl, azabicyclooctanyl, azabicyclononanyl, oxatricyclododecyl, azaadamantyl and oxaspiroheptanyl. The heterocyclyl can be optionally further substituted with 0, 1, 2, 3, 4 or 5 substituents selected from F, Cl, Br, I, =O, hydroxyl, -SR¹⁹, nitro, cyano, C₁₋₆ alkyl, C₁₋₆ hydroxyalkyl, C₁₋₆ alkoxy, C₂₋₆ alkenyl, C₂₋₆ alkynyl, C₃₋₈ carbocyclyl, 3- to 8-membered heterocyclyl, -(CH₂)ₖ-C(=O)-R¹⁹, -(CH₂)ₖ-C(=O)-O-R¹⁹, -(CH₂)ₖ-C(=O)-NR¹⁹R^{19a}, -(CH₂)ₖ-S(=O)ⱼ-R¹⁹, - O-C(=O)-O-R¹⁹ or -NR¹⁹R^{19a}. The heterocycle herein is as defined above.

"Optional" or "optionally" means that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

"Pharmaceutical composition" represents a mixture of one or more compounds described herein or a physiologically/pharmaceutically acceptable salt thereof or a stereoisomer, solvate, pharmaceutically acceptable salt or eutectic thereof and other components comprising physiologically/pharmaceutically acceptable carriers and/or excipients.

"Stereoisomer" refers to an isomer produced as a result of different spatial arrangement of atoms in molecules, including cis-trans isomers, enantiomers and conformational isomers.

"Effective dose" refers to an amount that causes a physiological or medical response in a tissue, system or subject and is a desirable amount, including the amount of a compound that is, when administered to a subject to be treated, sufficient to prevent occurrence of one or more symptoms of the disease or condition to be treated or to reduce the symptom(s) to a certain degree.

"Solvate" refers to the compound of the present invention or the salt thereof, and also includes stoichiometric or non-stoichiometric solvents bound by intermolecular non-covalent forces. When the solvent is water, the solvate is a hydrate.

### Detailed Description of Embodiments

The technical solutions of the present invention will be described in detail below in conjunction with the drawings and embodiments, but the scope of protection of the present invention includes but is not limited thereto.

### Synthesis example:

Compound 1: (2R)-N-[(1R)-1-(2-acetyl-2,7-diazaspiro[3.5]nonane-7-carbonyl)-5-amino-pentyl]-2-[[(2R)-2-[[(2R)-2-amino-3-phenyl-propionyl]amino]-3-phenylpropionyl]amino]-4-methyl-pentanamide (CAS number: 2269511-95-5) was prepared according to the method described in WO 2019015644.

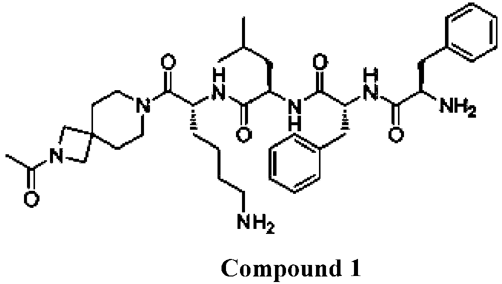

The remaining compounds 2-8 were prepared according to the methods described in WO 2019015644.

Formulation example: Compound 1 injection was prepared with reference to WO 2021036975 A1.

### Example 1:

This study was a multicentre, randomised, double-blind, placebo-controlled phase III clinical study conducted in China to evaluate the efficacy and safety of compound 1 injection for postoperative analgesia in subjects undergoing elective laparoscopic abdominal surgery under general anaesthesia. 276 subjects undergoing elective laparoscopic abdominal surgery under general anaesthesia were enrolled and randomised to two groups: the compound 1 group (136 subjects) and the placebo group (135 subjects). All subjects received an intravenous injection of compound 1 injection or placebo injection within 5 min after the completion of the first NRS score assessment, and the end time of the first postoperative administration was recorded as 0 min. At 8 h and 16 h after the end of the first administration, within a time window of ±15 min, a single corresponding dose of the compound 1 injection or placebo injection was administered intravenously to the subjects.

Resting NRS score assessments were performed in subjects within 5 min before the first postoperative administration, and at 15 min ± 5 min, 30 min ± 5 min, 1 h ± 5 min, 2 h ± 15 min, 4 h ± 15 min, 8 h (within 30 min before the second postoperative administration), 12 h ± 30 min, 16 h (within 30 min before the last postoperative administration), 20 h ± 30 min, and 24 h ± 1 h after the first postoperative administration. The NRS score for resting pain refers to the NRS score assessed when the subject is at rest (e.g., sitting or lying still). If the subject was unconscious at a scheduled NRS assessment time point, the score was recorded as follows: if the previous NRS score was ≥ 2, the current NRS score was recorded as 2; if the previous NRS score was < 2, the current NRS score was recorded as the previous score. Meanwhile, it was documented that the subject was unconscious at the time of assessment.

A morphine injection was administered via intravenous injection for rescue treatment if the resting pain NRS score was ≥ 4 at any pain assessment time point after the first postoperative administration, or if the subject complained of pain at any time point and the resting pain NRS score was ≥ 4. It was recommended that the time from the subject's NRS assessment to actual administration of morphine injection be ≤ 30 min; an initial intravenous dose of 3 mg be administered, followed by additional doses as needed. It was also recommended that the interval between two rescue treatments be ≥ 2 h and that the interval between rescue medication and study drug administration be ≥ 1 h.

Prophylactic antiemetic treatment was prohibited during the study. After the first postoperative administration, the investigator administered antiemetic agents based on the occurrence of nausea and vomiting in the subject, and the dosage of the antiemetic agents was recorded. Tropisetron was the preferred antiemetic agent (an initial intravenous dose of 2.5 mg, followed by additional doses as needed, not to exceed 10 mg within 24 h). If tropisetron was insufficiently effective, other agents were used by the investigator in accordance with routine clinical practice.

Primary efficacy results: The hypothesis that the SPID_{0-24 h} after the first postoperative administration in the compound 1 group was superior to that in the placebo group was confirmed. This indicates that compound 1 is superior to placebo for postoperative analgesia in subjects undergoing elective laparoscopic abdominal surgery under general anaesthesia.

Secondary efficacy results: The SPID_{0-12 h} after the first postoperative administration, PID at each time point, the mean cumulative dosage of rescue analgesic agents within 0-12 h and 0-24 h after the first postoperative administration, and the proportion of subjects using rescue analgesic agents within 0-24 h after the first postoperative administration in the compound 1 group were significantly lower than those in the placebo group. The duration of analgesia and the time to the first use of rescue analgesic agents in the compound 1 group were significantly longer than those in the placebo group. The proportion of subjects with NRS scores ≤ 3 within 0-12 h and 0-24 h after the first postoperative administration, and the subject and investigator satisfaction scores for postoperative analgesia in the compound 1 group were all significantly higher than those in the placebo group.

Safety results: The compound 1 group exhibited good overall safety and tolerability. The overall incidence of TEAEs (treatment-emergent adverse events) in the compound 1 group was similar to that in the placebo group, and the types of TEAEs were generally consistent between the two groups. Moreover, the incidences of the main TEAEs (including nausea and vomiting) in the compound 1 group were significantly lower than those in the placebo group. TEAEs leading to drug discontinuation and withdrawal from the study occurred only in the placebo group. The types of adverse reactions in the compound 1 group were generally consistent with those in the placebo group, and the incidences were comparable to those in the placebo group. Moreover, the incidences of adverse reactions (including nausea and vomiting) in the compound 1 group were significantly lower than those in the placebo group.

### Example 2:

This study was a multicentre, randomised, double-blind, placebo/active-controlled study. About 387 subjects undergoing elective abdominal surgery under general anaesthesia were planned to be enrolled and randomised to three groups: the compound 1 group (129 subjects), the tramadol group (129 subjects), and the placebo group (129 subjects).

After the end of surgery, MOAA/S scores were assessed every 5 ± 1 min. Once the subjects regained consciousness (defined as the first MOAA/S score of 5), the first resting pain intensity assessment was initiated within 5 min [using the numeric rating scale (NRS), where 0-10 represent varying degrees of pain: 0 represents no pain, higher numbers represent more severe pain, and 10 represents the most severe pain]. It was recommended that the interval between two NRS assessments not exceed 30 min. Within 4 h after the end of surgery, subjects who met all inclusion criteria and none of the exclusion criteria were randomised at a 1 : 1 : 1 ratio into the compound 1 group, the tramadol group or the placebo group. It was recommended that randomisation be performed within 10 min after the first NRS score ≥ 4.

After successful randomisation, to prevent the subjects from enduring pain for a long time, the first dose of compound 1 injection (1 µg/kg/dose), tramadol hydrochloride injection (50 mg/dose), or placebo injection was administered to the subjects by the investigator as soon as possible (recommended within 15 min after successful randomisation). The end time of the first postoperative administration of the study drug was recorded as 0 min. At 8 h (± 15 min) and 16 h (± 15 min) after the end of the first administration, a single corresponding dose of the compound 1 injection, tramadol hydrochloride injection, or placebo injection was administered to the subjects, for a total of three administrations.

Resting NRS score assessments were performed in subjects at 15 min ± 5 min, 30 min ± 5 min, 1 h ± 5 min, 2 h ± 15 min, 4 h ± 15 min, 8 h ± 30 min (within 30 min before the second administration of the study drug), 12 h ± 30 min, 16 h ± 30 min (within 30 min before the third administration of the study drug), 20 h ± 30 min, and 24 h ± 1 h after the end of the first administration of the study drug. The NRS score for resting pain refers to the NRS score assessed when the subject is at rest (e.g., sitting or lying still). If the subject was unconscious at a scheduled NRS assessment time point, the score was recorded as follows: if the previous NRS score was ≥ 2, the current NRS score was recorded as 2; if the previous NRS score was < 2, the current NRS score was recorded as the previous score. Meanwhile, it was documented that the subject was unconscious at the time of assessment.

A flurbiprofen axetil injection was administered via intravenous injection for rescue treatment if the subject complained of pain at any time point after the first administration of the study drug, and the resting pain NRS score was ≥ 4. It was recommended that the time from the subject's NRS assessment to actual administration of flurbiprofen axetil injection be ≤ 30 min; an initial intravenous dose of 50 mg be administered, followed by additional doses as needed. It was also recommended that the interval between two rescue treatments be ≥ 3 h and that the interval between rescue medication and study drug administration be ≥ 1 h. However, rescue treatment could be given at any time for severe pain. It was recommended that subjects who still failed to achieve pain control after use of rescue medication ≥ 4 times be withdrawn from the study at the investigator's discretion and receive other routine clinical analgesic treatments. Rescue details (including resting NRS score before rescue, the time of each use of rescue medication, the dosage of rescue medication, etc.) were recorded truthfully.

Prophylactic antiemetic treatment was prohibited during the study. After the first postoperative administration, the investigator administered antiemetic agents based on the occurrence of nausea and vomiting in the subject, and the dosage of the antiemetic agents was recorded. Tropisetron was the preferred antiemetic agent (it was recommended that an initial intravenous dose of 2.5 mg be administered, followed by additional doses as needed, not to exceed 10 mg within 24 h). If tropisetron was insufficiently effective, other agents were used by the investigator in accordance with routine clinical practice.

Primary efficacy results: The hypothesis that the SPID_{0-24 h} after the first postoperative administration in the compound 1 group was non-inferior to that in the tramadol injection group was confirmed. The hypothesis that the SPID₀₋₂₄ₕ in the compound 1 group after the first postoperative administration was superior to that in the placebo group was also confirmed. This indicates that compound 1 is non-inferior to tramadol injection and superior to placebo for postoperative analgesia in subjects undergoing elective abdominal surgery under general anaesthesia.

Secondary efficacy results: The SPID_{0-12 h} after the first postoperative administration, PID at each time point, the mean cumulative dosage of rescue analgesic agents within 0-12 h and 0-24 h after the first postoperative administration, and the proportion of subjects using rescue analgesic agents within 0-24 h after the first postoperative administration in the compound 1 group were comparable to those in the tramadol group and significantly lower than those in the placebo group. The duration of analgesia and the time to the first use of rescue analgesic agents in the compound 1 group were comparable to those in the tramadol group and significantly longer than those in the placebo group. The proportion of subjects with NRS scores ≤ 3 within 0-12 h and 0-24 h after the first postoperative administration, and the subject and investigator satisfaction scores for postoperative analgesia in the compound 1 group were generally comparable to those in the tramadol group and significantly higher than those in the placebo group.

Safety results: The compound 1 group exhibited good overall safety and tolerability. No serious adverse events occurred. The overall incidences of TEAEs (treatment-emergent adverse events) and ADRs (adverse drug reactions) in the compound 1 group were similar to those in the placebo group and significantly lower than those in the tramadol group. The majority of TEAEs/ADRs were Grade 1-2 and resolved either without intervention or after simple intervention. The types of TEAEs/ADRs were generally consistent across all groups. The incidence of decreased thyroid-stimulating hormone in the compound 1 group was slightly higher than that in the tramadol group and the placebo group; the incidences of nausea and vomiting were lower than those in the tramadol group and the placebo group. Meanwhile, the proportion of subjects using tropisetron and the cumulative dosage within 0-24 h after the first administration in the compound 1 group were significantly lower than those in the tramadol group and the placebo group; the proportion of subjects using other antiemetic agents within 0-24 h was slightly lower than those in the tramadol group and the placebo group, and the cumulative dosage was higher than those in the tramadol group and the placebo group.

Abdominal surgery, female sex, non-smoking status, and postoperative opioid use are known as risk factors leading to postoperation nausea and vomiting (PONV). Two studies of compound 1 injection for postoperative analgesia after abdominal surgery were completed. Morphine (Example 1) and flurbiprofen axetil (Example 2) were used as rescue analgesic agents in the two studies, respectively, and prophylactic antiemetic treatment was prohibited during the studies. The study results were summarised as follows.

In the safety analysis set of Example 1, the incidences of nausea during treatment were 17.6% and 27.4% in the compound 1 group and the placebo group, respectively; the incidences of vomiting were 15.4% and 32.6%, respectively. The proportion of subjects using antiemetic agents and the mean cumulative dosage of antiemetic agents within 0-24 h after the first administration in the compound 1 group were significantly lower than those in the placebo group.

Given that morphine was used as the rescue analgesic agent in this study, and patients might experience nausea and vomiting due to the use of rescue medication, we further analysed the occurrence of nausea and vomiting in subjects who did not receive rescue analgesia. The results showed that in the safety set, among subjects who did not receive rescue analgesia (no morphine use), the incidences of nausea and vomiting in the compound 1 group were lower than those in the placebo group, with reductions of 25.7% and 20.1% relative to the placebo group, respectively. Among patients who did not receive morphine for rescue in the study of Example 1, the difference between the Compound 1 group and the placebo group was smaller than that in the overall population, which may be related to the small sample size and large interindividual variability. However, most Phase III studies of PONV prevention require a relatively large sample size (e.g., > 500 subjects) to demonstrate the expected differences.

In Example 2, given that flurbiprofen axetil is a non-steroidal antiinflammatory drug and has not been identified as a cause of PONV in domestic or international guidelines, it is preliminarily considered that studies using flurbiprofen axetil as a rescue analgesic agent may better reflect the effect of compound 1 on PONV. In the safety analysis set, the incidences of nausea and vomiting in the compound 1 group were lower than those in the placebo group, with reductions of 50% and 47.9% relative to the placebo group, respectively.

### Comparison of incidence of postoperation nausea and vomiting between Example 1 and Example 2

| Adverse event | Example 1 | | | | Example 2 | |
|---|---|---|---|---|---|---|
| | Overall | | No morphine use | | | |
| | Compound 1 (N = 136) | Placebo (N = 135) | Compound 1 (N = 91) | Placebo (N = 72) | Compound 1 (N = 129) | Placebo (N = 129) |
| Nausea | 17.6% | 27.4% | 16.5% | 22.2% | 10.1% | 20.2% |
| Change in incidence of nausea relative to placebo | 35.8% reduction | / | 25.7% reduction | / | 50% reduction | / |
| Vomiting | 15.4% | 32.6% | 18.7% | 23.6% | 8.5% | 16.3% |
| Change in incidence of vomiting relative to placebo | 52.8% reduction | / | 20.1% reduction | / | 47.9% reduction | / |

In summary, both Phase III studies demonstrate that the compound 1 injection has a preventive and/or alleviating effect on PONV in patients after abdominal surgery.

### Example 3: Multicentre, randomised, double-blind, placebo-controlled dose study of compound 1 injection for the prevention of postoperation nausea and vomiting

**Study period:** Treatment duration: 24 h after surgery; Follow-up duration: 48 h after surgery

Protocol: This study was a multicentre, randomised, double-blind, placebo-controlled study. About 200 subjects undergoing elective laparoscopic abdominal or gynaecological surgery under general anaesthesia were planned to be enrolled and randomised at a 1 : 1 : 1 : 1 ratio into the compound 1-60 µg group (50 subjects), the compound 1-120 µg group (50 subjects), the compound 1-180 µg group (50 subjects), and the placebo group (50 subjects).

A single dose of the study drug was administered within 15 min before the end of surgery. The dosing regimen is as follows:
Compound 1-60 µg group: A single dose of 60 µg of compound 1 injection was administered within 15 min before the end of surgery, via intravenous bolus injection, with an intravenous bolus injection time of not less than 30 s.

Compound 1-120 µg group: A single dose of 120 µg of compound 1 injection was administered within 15 min before the end of surgery, via intravenous bolus injection, with an intravenous bolus injection time of not less than 30 s.

Compound 1-180 µg group: A single dose of 180 µg of compound 1 injection was administered within 15 min before the end of surgery, via intravenous bolus injection, with an intravenous bolus injection time of not less than 30 s.

Placebo group: A single dose of placebo was administered within 15 min before the end of surgery, via intravenous bolus injection, with an intravenous bolus injection time of not less than 30 s.

Primary efficacy endpoint: Complete response (CR) rate at 24 h after surgery (CR is defined as no vomiting or retching and no use of rescue medication within 24 h after surgery).

Secondary efficacy endpoints: Proportion of subjects experiencing nausea after surgery; proportion of subjects experiencing significant nausea after surgery (significant nausea is defined as a VAS score ≥ 4 cm); proportion of subjects experiencing vomiting after surgery (vomiting [even with expulsion of a minimal amount of gastric contents] or retching [muscular movement of vomiting without expulsion of gastric contents]); proportion of subjects using rescue medication after surgery; time to the first occurrence of emesis (vomiting or retching) or administration of rescue treatment within 24 h after surgery, whichever occurs first; cumulative dosage of analgesic agents within 24 h after surgery; and subject and investigator satisfaction scores for PONV.

Safety endpoints: Incidence and severity of adverse events; and vital signs, 12-lead ECG, physical examination, and laboratory examinations.

Screening period: From D-14 to successful randomisation (the day of randomisation is defined as D1).

Subjects planned to undergo laparoscopic abdominal or gynaecological surgery, who signed the informed consent form for this study, completed screening according to the trial flow chart. Subjects who successfully completed screening received a randomisation number before administration.

The method of anaesthesia for surgery was general anaesthesia, and the combination with other methods of anaesthesia (such as neuraxial block anaesthesia, nerve block anaesthesia, and local infiltration anaesthesia) was prohibited. Anaesthesia induction: Anaesthesia induction was recommended to be performed using propofol/cyclopropofol, sufentanil/remifentanil, and muscle relaxants. The use of midazolam was prohibited. Anaesthesia maintenance period: Anaesthesia maintenance was recommended to be performed using sevoflurane, remifentanil/sufentanil, and muscle relaxants. The use of propofol/cyclopropofol was prohibited. Other intraoperative medications were administered at the investigator's discretion.

Treatment period: From the start of study drug administration to 24 h after the end of surgery

Subjects who met all inclusion criteria and none of the exclusion criteria received a single dose of compound 1 injection or placebo within 15 min before the end of surgery (defined as the completion of the last suture of the wound).

The use of any antiemetic agents or agents with antiemetic effects, other than the study drug and rescue medications specified in the protocol, was prohibited from 24 h before the start of surgery (defined as the start of skin incision) to 24 h after the end of surgery. Prophylactic antiemetic treatment was prohibited.

Within 24 h after the end of surgery, if the subject experienced ≥ 1 episode of vomiting, retching, or nausea and complained of requiring antiemetic treatment, the subject received rescue antiemetic agents specified in the protocol after assessment by the investigator (tropisetron was the preferred rescue antiemetic agent; it was recommended that an initial intravenous dose of 2.5 mg be administered, followed by additional doses as needed, not to exceed 10 mg within 24 h). If tropisetron was insufficiently effective, other agents were used by the investigator in accordance with routine clinical practice.

Within 30 min after the end of surgery, a PCA pump containing morphine hydrochloride injection was initiated. Subjects self-administered analgesia by pressing the PCA pump when experiencing pain. PCA parameter settings: Concentration: 1 mg/ml; continuous infusion: 0 ml/L; PCA bolus of 2 ml per press, with a lockout interval of 10 min. If the patient still complained of pain, an additional dose of morphine hydrochloride injection was administered (a single intravenous dose of 3 mg) after assessment by the investigator.

The occurrence of vomiting and the occurrence and severity of any nausea in the subjects were monitored and recorded within 24 h after the end of surgery. Within 24 h after the end of surgery, any spontaneous nausea, retching, or vomiting symptoms were recorded by the subject, and the severity of nausea was assessed using a visual analogue scale (VAS scale, 0 cm = no nausea, 10 cm = the most severe unbearable nausea). The investigators inquired about and recorded the occurrence of nausea, retching, or vomiting in subjects in the preceding 12 h at 12 ± 1 h and 24 ± 1 h after surgery.

Subject and investigator satisfaction scores for PONV were assessed at 24 ± 1 h after the administration.

The dosage of rescue antiemetic agents and the cumulative dosage of analgesic agents administered to each subject within 24 h after surgery were recorded.

Follow-up period: From 24 h after the end of surgery to 48 h after the end of surgery

Vital signs, physical examination, 12-lead ECG, laboratory examinations, adverse events, and concomitant medications were collected from subjects. For subjects who had received the study drug but withdraw from the study early, every effort was made to complete the full set of pre-withdrawal examinations.

Inclusion criteria (subjects must meet all of the following criteria to be eligible for enrolment): 1. Age ≥ 18 years and ≤ 75 years, either gender; 2. American Society of Anesthesiologists (ASA) physical status Class I-III; 3. 18 kg/m² ≤ BMI ≤ 40 kg/m²; 4. Hospitalised subjects planned to undergo elective laparoscopic abdominal or gynaecological surgery under general anaesthesia, with an expected duration of anaesthesia maintenance ≥ 1 h; 5. Subjects assessed by the investigator as having a moderate or high risk (score ≥ 2) of postoperation nausea and vomiting (PONV) based on the Apfel simplified risk score; and 6. Consent to participate in this trial and voluntarily sign the informed consent form.

Exclusion criteria (subjects who meet any one of the following criteria will be excluded):

### Previous and concurrent medical conditions

1. Subjects with a history or evidence of any of the following diseases prior to screening: 1) Respiratory diseases: Severe chronic obstructive pulmonary disease, acute exacerbation of chronic obstructive pulmonary disease, severe airway stenosis, massive mass in the throat/larynx, history of (branch) tracheaoesophageal fistula or airway tear, or severe respiratory tract infection within 2 weeks prior to screening; 2) Central nervous system diseases: Epilepsy, Parkinson's disease, or other central nervous system diseases that cause nausea and vomiting, such as craniocerebral injury, intracranial space-occupying lesion, or intracranial aneurysm; 3) Cardiovascular diseases: Uncontrolled hypertension [systolic blood pressure (SBP) ≥ 170 mmHg and/or diastolic blood pressure (DBP) ≥ 105 mmHg without antihypertensive treatment, or SBP > 160 mmHg and/or DBP > 100 mmHg after antihypertensive treatment], or severe cardiac insufficiency (New York Heart Association [NYHA] Class III-IV), unstable angina pectoris, acute myocardial infarction, severe arrhythmia, history of tachycardia/bradycardia requiring drug therapy, second- to third-degree atrioventricular block (excluding those using a pacemaker) within 6 months prior to screening; 4) Digestive system diseases: Subjects with intestinal obstruction, or other digestive system diseases that are assessed by the investigator as likely to cause nausea and vomiting; 5) Definitively diagnosed vestibular dysfunction other than motion sickness (including but not limited to peripheral vestibular syndrome, central vestibular syndrome, etc.); and 6) A history of significant chronic dizziness.

### Previous and concurrent medications

2. Subjects who have received any of the following medications or treatments during the screening period:
1) Subjects who have received antiemetic agents or agents with antiemetic effects within 24 h before the start of surgery, or who have used antiemetic agents/agents with antiemetic effects within 5 half-lives before the start of surgery (whichever is longer); and 2) Subjects with malignant tumours who have received chemotherapy treatment within 4 weeks prior to screening.

### Laboratory and other examinations

3. Subjects whose laboratory examination parameters during the screening period meet any one of the following criteria: 1) White blood cell count < 3.0 × 10⁹/L; 2) Platelet count < 80 × 10⁹/L; 3) Haemoglobin < 70 g/L; 4) Prothrombin time (PT) prolonged by > 3 seconds above the upper limit of normal; 5) Activated partial thromboplastin time (APTT) prolonged by more than 10 seconds above the upper limit of normal; 6) Alanine aminotransferase (ALT) and/or aspartate aminotransferase (AST) > 3 × ULN; 7) Total bilirubin > 2 × ULN; 8) Serum creatinine > 2 × ULN; and 9) Fasting blood glucose ≥ 11.1 mmol/L.

### Other conditions

4. Subjects who are expected to require continued endotracheal intubation after surgery; 5. Subjects who are expected to require nasogastric or orogastric tube placement after surgery; 6. Subjects with a history of severe drug allergy, or who are allergic to the study drugs specified in the protocol; 7. Subjects with a history of drug abuse, substance abuse, or alcohol abuse within 3 months prior to screening, where alcohol abuse is defined as an average daily alcohol consumption > 2 alcohol units (1 unit = 360 mL of beer with 5% alcohol, 45 mL of spirits with 40% alcohol, or 150 mL of wine); 8. Subjects who experience nausea, retching, or vomiting within 24 hours prior to anaesthesia induction (excluding those caused by bowel preparation); 9. Subjects who have participated in any clinical study within 3 months prior to screening (defined as having received a study drug or placebo); 10. Female subjects who are pregnant or lactating; or subjects of reproductive potential (females or males) who are unwilling to use contraception throughout the study period and for 3 months after the end of the study; 11. Subjects assessed by the investigator as having any other condition that makes them unsuitable for participation in this clinical study.

Discontinuation criteria: 1. Subjects may withdraw consent and discontinue from the study at any time; 2. If any medical condition occurs before or during administration of the study drug that may pose a risk to the subject if treatment is continued, the investigator may decide to discontinue the subject's study drug therapy and/or withdraw the subject from the study; 3. Subjects who have a major protocol violation that poses a potential risk to subject safety and/or affects the objectivity and integrity of key study data shall be advised to withdraw from the study at the investigator's discretion; and 4. Any other circumstances in which the investigator deems it necessary for the subject to withdraw from the study.

Statistical analysis: Statistical analysis was performed using SAS EG version 8.3 or higher.

For continuous variables, the number of subjects, number of subjects with missing values, mean, standard deviation, median, quartiles (Q1 and Q3), minimum, and maximum were presented. For categorical variables, frequencies and percentages were described. In this study, unless otherwise stated, "baseline" is defined as the last non-missing observation data before the first administration of the study drug.

All analyses were performed separately by treatment group or dose group unless otherwise stated.

### Analysis datasets

Full anaysis set (FAS): According to the intention to treat (ITT) principle, the FAS includes all randomised subjects who received at least one dose of the study drug and had at least one efficacy assessment.

Per-protocol set (PPS): The PPS includes all subjects in the FAS who had good compliance during the study, complete data for the primary efficacy endpoint, and no major protocol deviations. Subjects with major protocol deviations to be excluded from the PPS were identified prior to database lock. A major protocol deviation refers to a protocol deviation that affects the primary efficacy endpoint.

Safety set (SS): The SS includes all randomised subjects who received at least one dose of the study drug and had at least one safety assessment after treatment.

### Efficacy analysis

Primary study endpoint: Statistical descriptions were made for the complete response (CR) rate at 24 h after surgery for each group. Logistic regression models were constructed with the complete response (CR) status at 24 h after surgery (CR is defined as no vomiting or retching and no use of rescue medication within 24 h after surgery), the proportion of subjects experiencing nausea within 24 h after surgery, the proportion of subjects experiencing significant nausea within 24 h after surgery, and the proportion of subjects experiencing vomiting within 24 h after surgery as dependent variables, and analysed with the actual baseline Apfel simplified risk score, cumulative postoperative morphine dosage, and treatment group as independent variables. Using the placebo group as the control, the odds ratio and the 95% confidence interval thereof for the complete response (CR) rate at 24 h after surgery in each treatment group were calculated, with the corresponding P-value provided. Relevant bar charts were generated.

### Secondary study endpoints:

Statistical descriptions were made for the proportion of subjects experiencing nausea within 24 h after surgery, the proportion of subjects experiencing significant nausea within 24 h after surgery, and the proportion of subjects experiencing vomiting within 24 h after surgery by treatment group. Logistic regression models were constructed with the proportion of subjects experiencing nausea within 24 h after surgery, the proportion of subjects experiencing significant nausea within 24 h after surgery, and the proportion of subjects experiencing vomiting within 24 h after surgery as dependent variables, and analysed with the actual baseline Apfel simplified risk score and treatment group as independent variables. Using the placebo group as the control, the odds ratio and the 95% confidence interval thereof for the percentage of subjects experiencing nausea within 24 h after surgery, the percentage of subjects experiencing significant nausea within 24 h after surgery, and the percentage of subjects experiencing vomiting within 24 h after surgery in each dose group of the study drug were calculated, with the corresponding P-value provided. Relevant bar charts were generated.

Statistical descriptions were made for the proportion of subjects using rescue medication within 24 h after surgery by treatment group. Between-group differences were analysed using the chi-square test or Fisher's exact test.

Statistical descriptions were made for the median time to the first occurrence of emesis (vomiting or retching) or administration of rescue treatment within 24 h after surgery (whichever occurs first) by treatment group. Kaplan-Meier curves were generated, and comparisons between each study drug group and the placebo group were performed using the Log-rank test.

Subject and investigator satisfaction scores for PONV: Descriptive statistics were made for the subject and investigator satisfaction scores for PONV by treatment group. Comparisons between groups were performed using the Kruskal-Wallis test, and comparisons between each dose group of the study drug and the placebo group were performed using the Wilcoxon rank-sum test.

### Safety analysis

### Adverse event:

Adverse events (AEs) were coded using the ICH Medical Dictionary for Regulatory Activities (MedDRA, Version 27.0 or higher) and classified by system organ class (SOC) and preferred term (PT). Unless otherwise specified, the severity of adverse events was graded according to the Common Terminology Criteria for Adverse Events (CTCAE V5.0). The number of subjects, incidence and number of episodes were calculated for all treatment-emergent adverse events (TEAEs), TEAEs of Grade 3 or higher, TEAEs related to the study drug, serious adverse events (SAEs), SAEs related to the study drug, and TEAEs leading to premature withdrawal of subjects from the study. The number of subjects and percentages were summarised by system organ class, preferred term and treatment group.

Specific information on AEs is presented in listings in detail. AEs occurring during the treatment after administration are described in detail.

### Examinations:

Statistical descriptions were made for laboratory examination parameters. Descriptive statistics were made for the results of laboratory examination parameters by baseline, each measurement time point after treatment, changes from baseline, and treatment group. Cross-tabulations were made for clinical assessments of laboratory examination parameters by baseline, each measurement time point after treatment, and treatment group. All results and clinically significant abnormal results are presented in listings.

Cross-tabulations were made for parameters such as 12-lead ECG, vital signs, and physical examination by baseline, each measurement time point after treatment, and treatment group. All results and clinically significant abnormal results are presented in listings.

Primary efficacy results: The complete response (CR) rate at 24 h after surgery in each dose group of compound 1 was higher than that in the placebo group, with statistically significant differences compared to the placebo group (P < 0.05). Among them, the 120 µg group exhibited the highest CR rate at 24 h after surgery.

Secondary efficacy results: The proportion of subjects experiencing nausea, severe nausea, vomiting, and using rescue medication within 24 h after surgery in each dose group of compound 1 was lower than that in the placebo group (see the table below for detailed data). The times to the first occurrence of emesis (vomiting or retching) or administration of rescue treatment within 24 h after surgery (mean ± standard deviation) were 11.6677 ± 10.6449 h, 18.3109 ± 9.6700 h, 20.1119 ± 8.4192 h, and 19.7203 ± 8.7225 h for the placebo group, the 60 µg group, the 120 µg group and the 180 µg group, respectively. The time to the first occurrence of emesis (vomiting or retching) or administration of rescue treatment within 24 h after surgery in each dose group of compound 1 was later than that in the placebo group (*P* < 0.05). The subject satisfaction scores for PONV (mean ± standard deviation) were 7.9 ± 2.27, 9.0 ± 1.89, 9.1 ± 1.52 and 9.0 ± 1.65 for the placebo group, the 60 µg group, the 120 µg group and the 180 µg group, respectively. The PONV satisfaction scores of subjects in each dose group of compound 1 were higher than those in the placebo group (P < 0.05). The investigator satisfaction scores for PONV were 7.9 ± 2.03, 8.9 ± 1.84, 8.9 ± 1.71 and 9.0 ± 1.39, respectively. The PONV satisfaction scores of investigators in each dose group of compound 1 were higher than those in the placebo group (P < 0.05).

### Efficacy results of Example 3

| | **60 µg group (N = 49) n (%)** | **120 µg group (N = 49) n (%)** | **180 µg group (N = 50) n (%)** | **Placebo group (N = 50) n (%)** |
|---|---|---|---|---|
| Complete response (CR) within 24 h after surgery | 36 (73.5) | 40 (81.6) | 39 (78.0) | 20 (40.0) |
| Nausea within 24 h after surgery | 18 (36.7) | 11 (22.4) | 16 (32.0) | 24 (48.0) |
| Significant nausea within 24 h after surgery | 9 (18.4) | 7 (14.3) | 11 (22.0) | 16 (32.0) |
| Vomiting within 24 h after surgery | 12 (24.5) | 8 (16.3) | 9 (18.0) | 27 (54.0) |
| Use of rescue medication within 24 h after surgery | 7 (14.3) | 6 (12.2) | 8 (16.0) | 19 (38.0) |

The safety results suggested that: Compound 1 exhibited good overall safety. The overall incidence of TEAEs was lower than that in the placebo group, while the incidence of ADRs was slightly higher than that in the placebo group. All TEAEs (treatment-emergent adverse events) and ADRs (adverse drug reactions) were Grade 1-2. No TESAEs (treatment-emergent serious adverse events) occurred, and no TEAEs leading to study withdrawal or death were observed. The TEAEs and ADRs occurring in this study were generally consistent with those in previous studies. No new adverse events or new safety signals were observed.

### Example 4: Multicentre, randomised, double-blind, active-controlled dose-finding study of compound 1 injection for the prevention of chemotherapy-induced nausea and vomiting (CINV)

**Study period:** Treatment duration: Within 120 h after the first administration of highly emetogenic chemotherapeutic agents; Follow-up duration: Day 10 ± 1 after the first administration of highly emetogenic chemotherapeutic agents.

**Protocol:** This study was a multicentre, randomised, double-blind, active-controlled study. About 180 tumour subjects planned to receive highly emetogenic chemotherapy for the first time were randomised in a 1 : 1 : 1 ratio to three groups, with 60 subjects per group. Pre-chemotherapy study drug administration was completed 30 ± 10 min before the start of administration of the highly emetogenic chemotherapeutic agents. The dosing regimen was as follows:
administration was performed in the following order:
**1) Compound 1 (placebo)/compound 1:** A single dose was administered 30 ± 10 min before the start of administration of highly emetogenic chemotherapeutic agents, via intravenous bolus injection, with an intravenous bolus injection time of not less than 30 s.
**2) Dolasetron mesylate injection/dolasetron mesylate injection matching placebo:** After the administration of compound 1/compound 1 (placebo), 5 ml (100 mg) of dolasetron mesylate injection/matching placebo was administered via intravenous bolus injection without dilution, and the infusion line was flushed with normal saline before and after administration. The intravenous bolus injection was performed over no less than 30 s.
**3) Aprepitant injection:** After the administration of dolasetron mesylate injection/matching placebo, 18 mL (130 mg) of aprepitant solution was administered via intravenous bolus injection without dilution, and the infusion line was flushed with normal saline before and after administration. The intravenous bolus injection was performed over no less than 2 min.
**4) Dexamethasone tablets:** 6 mg of dexamethasone was orally administered at 30 ± 10 min before the start of administration of highly emetogenic chemotherapeutic agents. On D2 to D4, 3.75 mg of dexamethasone was administered orally QD.

### Primary efficacy endpoint:

- complete response (CR) rate at 24 h (acute phase) after administration of highly emetogenic chemotherapeutic agents (CR is defined as no vomiting or retching and no use of rescue medication within 24 h after chemotherapy).

*Vomiting: even with expulsion of a minimal amount of gastric contents; Retching: muscular movements of vomiting without expulsion of gastric contents.*

### Secondary efficacy endpoints:

- complete response (CR) rates at 0-120 h (overall phase) and 24-120 h (delayed phase) after administration of highly emetogenic chemotherapeutic agents;
- proportion of subjects with no nausea (nausea VAS score ≤ 5 mm) and mild nausea (nausea VAS score ≤ 25 mm) at 0-24 h and 24-120 h after administration of highly emetogenic chemotherapeutic agents;
- proportion of subjects with no vomiting at 0-24 h and 24-120 h after administration of highly emetogenic chemotherapeutic agents;
- proportion of subjects using rescue medication at 0-24 h and 24-120 h after administration of highly emetogenic chemotherapeutic agents;
- time to the first occurrence of emesis (vomiting or retching) or administration of rescue treatment within 120 h after administration of highly emetogenic chemotherapeutic agents, whichever occurs first;
- mean VAS scores at 24 ± 1 h and 120 ± 1 h after administration of highly emetogenic chemotherapeutic agents;
- functional living index-emesis (FLIE) score;
- subject and investigator satisfaction scores for CINV prevention.

### Safety endpoints:

- incidence and severity of adverse events;
- vital signs, 12-lead ECG, physical examination, laboratory examinations, etc.

### Pharmacokinetic endpoint:

plasma concentrations of compound 1 at predetermined time points.

Screening period: D-14 to D-1 (the day of administration of highly emetogenic chemotherapeutic agents was recorded as D1)

Subjects planned to receive highly emetogenic chemotherapy for the first time signed the informed consent form, completed screening period examinations, and had the inclusion and exclusion criteria checked.

Before administration of highly emetogenic chemotherapeutic agents, subjects who had been assigned a random number received training on study-related scales, including recording of post-chemotherapy nausea and vomiting, assessment of nausea severity using the visual analogue scale (VAS), rescue medication record form, satisfaction assessment form, functional living index-emesis (FLIE), etc.

Treatment and observation period: D1 to D5 (from the day of start of study drug administration to 120 h after start of highly emetogenic chemotherapeutic agents)

Before study drug administration, the inclusion and exclusion criteria were checked. Subjects who met all inclusion criteria and none of the exclusion criteria were randomised at a 1 : 1 : 1 ratio to group A, group B or group C, were assigned a random number, and received different CINV prophylaxis regimens. Pre-chemotherapy study drug administration was completed 30 ± 10 min before the start of highly emetogenic chemotherapy. See the dosing regimen for details. Prophylactic antiemetic agents were administered on D1 to D4.

The time of start of administration of highly emetogenic chemotherapeutic agents is defined as 0 min.

On D1, 12-lead ECG examinations were performed within 30 min before administration of compound 1/compound 1 (placebo), and at 30 s to 5 min and 1 h ± 10 min after administration of compound 1/compound 1 (placebo).

Within 120 h after the start of administration of highly emetogenic chemotherapeutic agents, the subject was required to record the occurrence of nausea and vomiting after chemotherapy and filled in the post-chemotherapy nausea and vomiting record form when vomiting, retching or nausea occurred at any time point. Within 2 ± 0.5 h, 4 ± 1 h, 8 ± 1 h, and 12 to 24 h after the start of administration of highly emetogenic chemotherapeutic agents, the investigator inquired about the occurrence of vomiting, retching, and nausea during the corresponding time intervals, verified the completeness of the subject's records, and supplemented any missing entries if necessary. Thereafter, the investigator inquired about the occurrence of vomiting, retching, and nausea during the corresponding time intervals every 24 ± 2 h, verified the completeness of the subject's records, and supplemented any missing entries if necessary. If the subject had been discharged from the hospital, telephone follow-up was performed.

At any time when the subject complained of nausea, the severity of nausea was assessed using a 100 mm visual analogue scale (VAS), and the score was recorded in the post-chemotherapy nausea and vomiting record form. A VAS score ≤ 5 mm was considered no nausea and a VAS score ≤ 25 mm was considered mild nausea.

If the subject experienced ≥ 1 episode of vomiting, retching, or nausea at any time and complained of requiring antiemetic therapy, rescue antiemetic treatment was administered after assessment by the investigator. The antiemetic agent was administered at the investigator's discretion. If rescue medication was administered while the subject was in the hospital, the investigator recorded the use of rescue medication. If the subject had been discharged from the hospital, the subject self-administered antiemetic agents. The investigator confirmed whether the subject received rescue medication during the visit, verified the completeness of the subject's records, and supplemented any missing entries if necessary.

At 24 ± 1 h and 120 ± 1 h after the start of administration of highly emetogenic chemotherapeutic agents, the subject was required to complete the subject satisfaction score scale for CINV prevention and the functional living index-emesis (FLIE) scale. The investigator was required to confirm with the subjects that the corresponding scale had been completed, and to complete the investigator satisfaction score scale (if the subjects had been discharged from the hospital, the scale was submitted to the investigator for review during the return follow-up visit).

Subjects were randomised to the PK subgroup. A total of 90 subjects were planned to be enrolled in the PK subgroup for PK blood collection. Subjects enrolled in the PK subgroup were randomised at a 1 : 1 : 1 ratio to groups A, B and C (30 subjects per group). Venous blood was collected for PK analysis at 4 time points: immediately (+ 5 min), 2 h (± 1 h), 6 h (± 2h), and 12 h (± 2h) after the end of administration of compound 1/compound 1 (placebo). PK blood sampling was not performed on the same arm as the administration vein. When the PK blood collection time point conflicted with the ECG examination time, ECG examination was performed first.

During the treatment and observation period, the daily antitumour treatment regimen received by the subjects was recorded, including the use of antitumour drugs and non-pharmacological treatments.

### Follow-up period: D6 to D10 ± 1

During D6 to D8 after the start of administration of highly emetogenic chemotherapeutic agents: Subjects returned to the hospital for follow-up. The post-chemotherapy nausea and vomiting record forms, rescue medication record forms, subject satisfaction scales and FLIE scales completed by the subjects during the treatment and observation period were collected and checked against the investigator's visit inquiry records to confirm accuracy.

Vital signs, physical examination, 12-ECG, laboratory examinations, pregnancy test, adverse events, and concomitant medications/treatments (including antitumour treatment regimens on and after D6) were collected from subjects, and relevant data were recorded in the study medical records and EDC system.

D10 ± 1 (telephone follow-up is acceptable): Adverse events and concomitant medications/treatments were collected from the subjects.

For subjects who had received the study drug but had not completed the follow-up period (D6 to D8) examinations and withdraw from the study early, every effort was made to complete the full set of pre-withdrawal examinations (identical to those required at D6 to D8) and to collect information on AEs and concomitant medications/treatments.

### Inclusion criteria (subjects must meet all of the following criteria to be eligible for enrolment):

1. age ≥ 18 years, either gender;
2. no previous exposure to chemotherapeutic agents, and planned to receive a single-day intravenous highly emetogenic chemotherapy regimen in this study, including but not limited to AC combination regimens (all chemotherapy regimens containing anthracyclines and cyclophosphamide), carboplatin AUC ≥ 4, carmustine > 250 mg/m², cisplatin, cyclophosphamide > 1500 mg/m², dacarbazine, doxorubicin ≥ 60 mg/m², ifosfamide ≥ 2 g/m² (single dose), mechlorethamine, sacituzumab govitecan and streptozocin;
3. histologically or cytologically confirmed malignant solid tumour;
4. Eastern Cooperative Oncology Group (ECOG) performance status score of 0 or 1;
5. life expectancy ≥ 3 months;
6. subjects have adequate bone marrow, renal and hepatic function:
   a) absolute neutrophil count ≥ 1.5 × 10⁹/L, white blood cell count ≥ 3.0 × 10⁹/L;
   b) platelet count ≥ 75 × 10⁹/L;
   c) haemoglobin ≥ 70 g/L;
   d) aspartate aminotransferase (AST) ≤ 3 × ULN (≤ 5 × ULN for patients with hepatocellular carcinoma or liver metastasis);
   e) alanine aminotransferase (ALT) ≤ 3 × ULN (≤ 5 × ULN for patients with hepatocellular carcinoma or liver metastasis);
   f) serum total bilirubin ≤ 2 × ULN (≤ 3 × ULN for patients with hepatocellular carcinoma or liver metastasis);
   g) creatinine ≤ 2 × ULN;
7. consent to participate in this trial and voluntarily sign the informed consent form.

Exclusion criteria (subjects who meet any one of the following criteria will be excluded):
1. subjects with any of the following current medical conditions prior to screening:
   a) primary or metastatic central nervous system malignancy;
   b) epilepsy, Parkinson's disease, or other central nervous system diseases that cause nausea and vomiting;
   c) intestinal obstruction, or other digestive system diseases that are assessed by the investigator as likely to cause nausea and vomiting;
   d) definitively diagnosed vestibular dysfunction other than motion sickness (including but not limited to peripheral vestibular syndrome and central vestibular syndrome);
   e) a history of significant chronic dizziness;
   f) QT interval > 450 ms at screening, or use of concomitant medications for prolonged QT interval, or risk factors for prolonged QT interval or corresponding arrhythmia events (heart failure, hyperkalaemia, or history or family history of long QT syndrome);
2. subjects with known allergy or contraindication to the study drug specified in the protocol or other study drugs (including chemotherapeutic agents, components of the study drug and matching placebo, dolasetron, aprepitant, dexamethasone, etc.);
3. subjects who have experienced nausea, retching, or vomiting within 24 h prior to randomisation;
4. subjects who have received abdominal or pelvic radiotherapy within 7 days prior to randomisation, or plan to receive such radiotherapy during the study period;
5. subjects who plan to receive another dose of highly emetogenic chemotherapeutic agents within 5 days after the administration of the highly emetogenic chemotherapeutic agents, or plan to receive highly emetogenic chemotherapeutic agents for multiple days in one chemotherapy cycle;
6. subjects who have received antiemetic agents/agents with antiemetic effects within 5 half-lives prior to chemotherapy, including but not limited to 5-HT₃ receptor antagonists (such as tropisetron, ondansetron), NK-1 receptor antagonists (such as aprepitant and fosaprepitant), systemic corticosteroids (such as dexamethasone and methylprednisolone), antidopaminergic agents (such as amisulpride and droperidol), antihistamines (such as diphenhydramine and promethazine), etc.;
7. subjects who have received other medications that affect the assessment of nausea and vomiting within 5 half-lives prior to chemotherapy, or plan to receive such medications during the study period, including but not limited to benzodiazepines, opioids and systemic glucocorticoids;
8. subjects who received specific CYP3A4 substrates, CYP3A4 inhibitors, or inducers within 5 half-lives prior to chemotherapy, or plan to receive such agents during the study period;
9. subjects who have participated in any clinical study within 1 month prior to screening (defined as having received a study drug or placebo);
10. subjects with a history of drug abuse, substance abuse or alcohol abuse within 3 months prior to screening, where alcohol abuse is defined as an average daily alcohol consumption > 2 alcohol units (1 unit = 360 mL of beer with 5% alcohol, 45 mL of spirits with 40% alcohol, or 150 mL of wine);
11. subjects with active hepatitis B (positive HBsAg and HBV-DNA above the upper limit of normal), positive hepatitis C antibody test, syphilis infection (positive anti-TP test), or human immunodeficiency virus (HIV) infection (positive anti-HIV test) during the screening period;
12. female subjects who are pregnant or lactating; or subjects of reproductive potential (females or males) who are unwilling to use contraception throughout the study period and for 3 months after the last administration of study drug;
13. subjects assessed by the investigator as having any other condition that makes them unsuitable for participation in this clinical study.

### Discontinuation criteria:

1. During the study, the subject is found to have violated the inclusion/exclusion criteria at enrolment, which, at the investigator's discretion, compromises the subject's safety.
2. The subject withdraws informed consent.
3. AE occurs that renders the subject unsuitable to continue study treatment, at the investigator's discretion.
4. Unblinding of the study drug.
5. Pregnancy.
6. Serious non-compliance with protocol-specified procedures, including study treatment, which, at the investigator's discretion, compromises safety assessment or efficacy analysis.
7. Loss to follow-up.
8. Other reasons.

### Statistical analysis:

Statistical analysis was performed using SAS EG version 8.3 or higher.

For continuous variables, the number of subjects, number of subjects with missing values, mean, standard deviation, median, quartiles (Q1 and Q3), minimum, and maximum were presented. For categorical variables, frequencies and percentages were described. In this study, unless otherwise stated, "baseline" is defined as the last non-missing observation data before the first administration of the study drug.

All analyses were performed separately by treatment group unless otherwise stated.

### • Analysis datasets

Full anaysis set (FAS): According to the intention to treat (ITT) principle, the FAS includes all randomised subjects who received at least one dose of the study drug and had at least one efficacy assessment.

Per-protocol set (PPS): The PPS includes all subjects in the FAS who had good compliance during the study, complete data for the primary efficacy endpoint, and no major protocol deviations. Subjects with major protocol deviations to be excluded from the PPS were identified prior to database lock. A major protocol deviation refers to a protocol deviation that affects the primary efficacy endpoint.

Safety set (SS): The SS includes all randomised subjects who received at least one dose of the study drug and had at least one safety assessment after treatment.

Pharmacokinetics analysis set (PKS): The PKS includes all randomised subjects who received at least one dose of the study drug, had at least one measurable plasma concentration, and had no protocol deviations that affect pharmacokinetic data.

### Efficacy analysis

**Primary study endpoint:** Statistical descriptions were made for the complete response (CR) rate at 24 h after administration of highly emetogenic chemotherapeutic agents for each group. The difference in the complete response (CR) rates at 24 h after administration of highly emetogenic chemotherapeutic agents was analysed by treatment group using the Chi-square test or Fisher's exact test (CR is defined as no vomiting or retching and no use of rescue medication within 24 h after administration of highly emetogenic chemotherapeutic agents).

### Secondary study endpoints:

Statistical descriptions were made for the CR rate at 0-120 h (overall phase) and 24-120 h (delayed phase) after administration of highly emetogenic chemotherapeutic agents, the proportion of subjects without nausea at 0-24 h and 24-120 h after administration of highly emetogenic chemotherapeutic agents, the proportion of subjects without vomiting at 0-24 h and 24-120 h after administration of highly emetogenic chemotherapeutic agents, and the proportion of subjects using rescue medication at 0-24 h and 24-120 h after administration of highly emetogenic chemotherapeutic agents, as well as the mean VAS score at 24 ± 1 h and 120 ± 1 h after administration of highly emetogenic chemotherapeutic agents, by treatment group. Between-group comparisons of quantitative variables were performed using analysis of variance or nonparametric tests, and differences in qualitative variables were analysed using the Chi-square test or Fisher's exact test.

Statistical descriptions were made for the median time to the first occurrence of emesis (vomiting or retching) or administration of rescue treatment within 120 h after administration of highly emetogenic chemotherapeutic agents (whichever occurs first) by treatment group. Kaplan-Meier curves were generated, and between-group comparisons among the study drug groups were performed using the Log-rank test.

Subject and investigator satisfaction scores for CINV prevention and functional living index-emesis (FLIE): Descriptive statistics were made for the subject and investigator satisfaction scores and functional living index-emesis (FLIE) by treatment group. Between-group comparisons of satisfaction scores were performed using the nonparametric Wilcoxon rank-sum test.

### Safety analysis

### Adverse event:

Adverse events (AEs) were coded using the ICH Medical Dictionary for Regulatory Activities (MedDRA, Version 27.0 or higher) and classified by system organ class (SOC) and preferred term (PT). Unless otherwise specified, the severity of adverse events was graded according to the Common Terminology Criteria for Adverse Events (CTCAE) [version 5.0]. The number of subjects, incidence and number of episodes were calculated for all treatment-emergent adverse events (TEAEs), TEAEs of Grade 3 or higher, TEAEs related to the study drug, serious adverse events (SAEs), SAEs related to the study drug, and TEAEs leading to premature withdrawal of subjects from the study. The number of subjects and percentages were summarised by system organ class, preferred term and treatment group.

Specific information on AEs is presented in listings in detail. AEs occurring during the treatment after administration are described in detail.

### Examinations:

Statistical descriptions were made for laboratory examination parameters. Descriptive statistics were made for the results of laboratory examination parameters by baseline, each measurement time point after treatment, changes from baseline, and treatment group. Cross-tabulations were made for clinical assessments of laboratory examination parameters by baseline, each measurement time point after treatment, and treatment group. All results and clinically significant abnormal results are presented in listings.

Cross-tabulations were made for parameters such as 12-lead ECG, vital signs, and physical examination by baseline, each measurement time point after treatment, and treatment group. All results and clinically significant abnormal results are presented in listings.

### Pharmacokinetic parameters:

Descriptive statistical analysis was made for plasma concentrations of compound 1 by time point and treatment group.

Plasma concentration data from this study were combined with data from other clinical trials of compound 1 to establish a population pharmacokinetic model. This model was used to evaluate the effect of covariates on the pharmacokinetic characteristics of compound 1. In addition, exposure-response analyses were performed for specific efficacy and safety endpoints. The results of the above population pharmacokinetic and exposure-response analyses were documented in a separate report.

The results of the study in this example indicated that compound 1 injection has a preventive and/or ameliorating effect on CINV, with good overall safety.

## Claims

1. Use of a compound of formula (I) or a stereoisomer, hydrate, metabolite, solvate, pharmaceutically acceptable salt or eutectic thereof, and a composition thereof in the preparation of a drug for preventing postoperation nausea and vomiting or chemotherapy-induced nausea and vomiting: 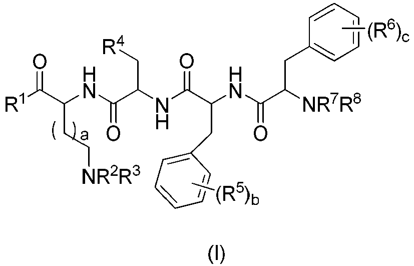 **characterized in that**
R₁ is selected from 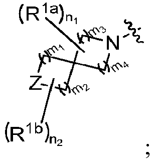
m1, m2, m3 and m4 are each independently selected from 0, 1, 2, 3 or 4; m1 and m2 are not both 0; m3 and m4 are not both 0;
n1 and n2 are each independently selected from 0, 1, 2, 3 or 4;
Z is selected from CR^{zl}R^{z2} or NR^{z3};
R^{z1} and R^{z2} are each independently selected from H, F, Cl, Br, I, OH, CF₃, nitro, C₁₋₆ alkyl, C₁₋₆ alkoxy, C₂₋₆ alkenyl, C₂₋₆ alkynyl, -C(=O)-C₁₋₆ alkyl, -(CH₂)_{q}-C(=O)O-C₁₋₆ alkyl, -(CH₂)_{q}-NR^{le}R^{lf}, -(CH₂)_{q}-COOH, -(CH₂)_{q}-CONH₂, C₃₋₈ carbocyclyl or 3- to 8-membered heterocyclyl, wherein the alkyl, the alkoxy, the alkenyl, the alkynyl, the carbocyclyl or the heterocyclyl is optionally further substituted with 0-5 substituents selected from F, Cl, Br, I, OH, CF₃, =O, carboxyl, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, the heterocyclyl contains 1 to 3 heteroatoms optionally selected from N, O or S, and when the heteroatom is selected from S, the heterocyclyl is optionally further S, S=O or S(=O)₂;
R^{le} and R^{lf} are each independently selected from H, C₁₋₆ alkyl, C(=O)O-C₁₋₆ alkyl, -C(=O)O-(CH₂)q-C₃₋₈ carbocyclyl or -C(=O)O-(CH₂)_{q}-3- to 8-membered heterocyclyl, wherein the alkyl, the carbocyclyl or the heterocyclyl is optionally further substituted with 0-5 substituents selected from F, Cl, Br, I, OH, CF₃, cyano, nitro, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms selected from N, O or S;
or R^{z1} and R^{z2}, together with the carbon atom to which they are attached, form a 3- to 10-membered nitrogen-containing heterocyclic ring, wherein the ring is optionally further substituted with a substituent selected from F, Cl, Br, I, OH, CF₃, cyano, nitro, =O, C₁₋₆ alkyl, C₁₋₆ alkoxy, C₂₋₆ alkenyl, C₂₋₆ alkynyl, C_{.} carbocyclyl or 3- to 8-membered heterocyclyl;
R^{la} and R^{lb} are each independently selected from F, CF₃, NH₂, C₁₋₆ alkyl, C₂₋₆ alkenyl, C₂₋₆ alkynyl or 3- to 8-membered heterocyclyl, wherein the alkyl, the alkenyl, the alkynyl or the heterocyclyl is optionally further substituted with 0-5 substituents selected from F, Cl, Br, I, OH, CF₃, nitro, cyano, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
R^{z3} is independently selected from H, -C(=O)-C₁₋₆ alkyl, -C(=O)O-C₁₋₆ alkyl, -C(=O)-C₃₋₈ carbocyclyl, -C(=O)O-C₃₋₈ carbocyclyl, -C(=O)O-(3- to 8-membered heterocyclyl), -S(=O)ₚ-C₁₋₆ alkyl, -S(=O)ₚ-C₃₋₈ carbocyclyl, -S(=O)ₚ-(3- to 8-membered heterocyclyl), -C(=O)NR^{1g}R^{lh}, -S(=O)ₚ-NR^{li}R^{lj} or 3- to 8-membered heterocyclyl, wherein the alkyl, the carbocyclyl or the heterocyclyl is optionally further substituted with 0-5 substituents selected from F, Cl, Br, I, OH, CF₃, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
R^{lg}, R^{lh}, R^{li'} and R^{lj} are each independently selected from H or C₁₋₆ alkyl;
or R^{lg} and R^{lh}, together with the nitrogen atoms to which they are attached, form a 3- to 10-membered heterocyclic ring, wherein the ring is optionally further substituted with a substituent selected from F, Cl, Br, I, OH, CF₃, cyano, nitro, amino, C₁₋₆ alkyl, C₁₋₆ alkoxy, C₂₋₆ alkenyl, C₂₋₆ alkynyl or -S(=O)ₚ-C₁₋₆ alkyl, and the heterocyclyl contains 1 to 3 heteroatoms selected from N, O or S;
q is selected from 0, 1, 2, 3 or 4;
p is selected from 0, 1 or 2;
a is selected from 0, 1, 2 or 3;
R⁴ is independently selected from H, C₁₋₆ alkyl, C₂₋₆ alkenyl, C₂₋₆ alkynyl or - (CH₂)q-C₃₋₈ carbocyclyl, wherein the alkyl, the alkenyl, the alkynyl or the carbocyclyl is optionally further substituted with 0-5 substituents selected from F, Cl, Br, I, OH, CN, CF₃, NO₂, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms selected from N, O or S;
R², R³, R⁷ and R⁸ are each independently selected from H, C₁₋₆ alkyl, - C(=O)O-C₁₋₄ alkyl, -C(=O)O-(CH₂)_{q}-C₃₋₈ carbocyclyl, -C(=O)O-(CH₂)_{q}-3- to 8-membered heterocyclyl or 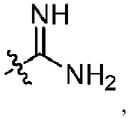 wherein the alkyl, the carbocyclyl or the heterocyclyl is optionally further substituted with 0-5 substituents selected from F, Cl, Br, I, OH, CF₃, nitro, cyano, amino, C₁₋₆ alkyl, C₁₋₆ alkoxy, C₂₋₆ alkenyl, C₂₋₆ alkynyl, C₃₋₈ carbocyclyl or 3- to 8-membered heterocyclyl, and the heterocyclyl contains 1 to 3 heteroatoms optionally selected from N, O or S;
b is selected from 0, 1, 2, 3, 4 or 5;
c is selected from 0, 1, 2, 3, 4 or 5;
R⁵ and R⁶ are each independently selected from F, Cl, Br, I, OH, CN, CF₃, cyano, nitro, C₁₋₄ alkyl, -OR^{5a}, -C(O)OR^{5b}, -SR^{5c}, -S(O)R^{5d}, -S(O)₂R^{5e} or -NR^{5f}R^{5g};
R^{5a}, R^{5b}, R^{5c}, R^{5d}, R^{5e}, R^{5f} and R^{5g} are each independently selected from H or C1-4 alkyl;
or R^{5f} and R^{5g}, together with the nitrogen atom to which they are attached, form a 5- to 6-membered heterocyclic ring, wherein the heterocyclyl contains 1 to 3 heteroatoms optionally selected from N, O or S.

2. The use according to claim 1, **characterized in that** the compound is selected from formula (II): 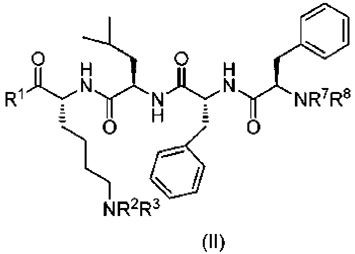 wherein
R₁ is selected from 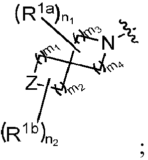
m1, m2, m3 and m4 are each independently selected from 0, 1 or 2; m1 and m2 are not both 0; m3 and m4 are not both 0;
n1 and n2 are each independently selected from 0 or 2;
Z is selected from CR^{z1}R^{z2} or NR^{z3};
R^{1a} and R^{1b} are independently selected from F or NH₂;
R^{z1} and R^{z2} are each independently selected from H, carboxyl, 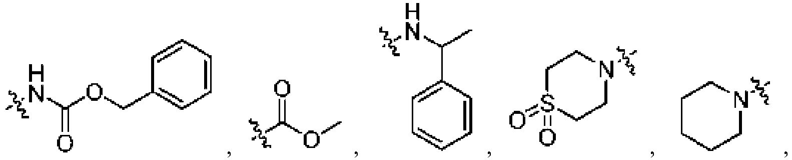 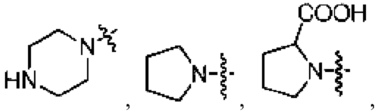 amino, -CH₂NH₂ or 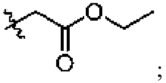
or R^{z1} and R^{z2} can, together with the carbon atom to which they are attached, form a lactam 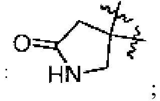
R^{z3} is independently selected from H, -C(=O)-C₁₋₄ alkyl, -C(=O)O-C₁₋₄ alkyl, -C(=O)-C₃₋₆ carbocyclyl, -C(=O)O-C₃₋₆ carbocyclyl, -S(=O)ₚ-C₁₋₄ alkyl, -S(=O)ₚ-C₃₋₆ carbocyclyl, -C(=O)NR^{1g}R^{1h}, -S(=O)ₚ-NR¹ⁱR^{1j} or 3- to 6-membered heterocyclyl, wherein the alkyl, the carbocyclyl or the heterocyclyl is optionally further substituted with 0-5 substituents selected from F, Cl, Br, I, OH, CF₃, nitro, cyano, amino, methyl, ethyl, methoxy, ethoxy, cyclopropyl or phenyl, and the heterocyclyl contains 1 to 3 heteroatoms optionally selected from N, O or S;
R^{lg}, R^{lh}, R^{li'} and R^{lj} are each independently selected from H or C₁₋₄ alkyl;
or R^{lg} and R^{lh}, together with the nitrogen atom to which they are attached, form a 4- to 6-membered heterocyclic ring, wherein the ring is optionally further substituted with a substituent selected from F, CF₃, methyl, methoxy or -S(=O)ₚ-C₁₋₄ alkyl, and the heterocyclyl contains 1 to 3 heteroatoms selected from N, O or S;
p is selected from 2;
R², R³, R⁷ and R⁸ are each independently selected from H, methyl or C(=O)O-tert-butyl.

3. The use according to claim 2, **characterized in that** the compound of formula (II) is selected from:

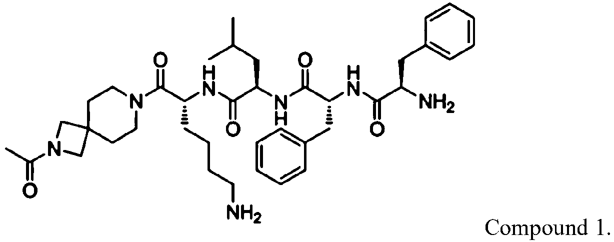

4. The use according to claim 3, **characterized in that** the pharmaceutically acceptable salt is selected from propionate, mesylate, acetate, citrate, D-tartrate, besylate, phosphate, aspartate, L-tartrate, maleate, fumarate, benzoate, lactate, hydrochloride, formate, hydrobromide, sulphate, nitrate, phosphate, trifluoroacetate, succinate, mandelate, malonate, malate, 2-hydroxypropionate, oxalate, glycolate, salicylate, citrate, glutamate, cinnamate, p-toluenesulphonate, benzenesulphonate, ethanesulphonate or triflate.

5. The use according to claim 4, **characterized in that** an administration form is one or more of single administration, multiple administrations, continuous administration, and target-controlled infusion.

6. The use according to claim 4, **characterized in that** a route of administration is selected from oral administration, injection, infusion, transdermal absorption, buccal absorption, parenteral intraperitoneal administration, rectal administration, transbuccal administration, intranasal administration, inhalation, topical delivery, subcutaneous administration, intraadipose administration, intraarticular administration, intraperitoneal administration or intrathecal administration; preferably intravenous injection, intravenous infusion, intraarterial injection, intramuscular injection, subcutaneous injection, intraarticular injection, intraperitoneal injection, intrathecal injection or intranasal administration.

7. The use according to claim 6, **characterized in that** the compound of formula (I) or the stereoisomer, hydrate, metabolite, solvate, pharmaceutically acceptable salt or eutectic thereof, and the composition thereof are administered before the end of surgery.

8. The use according to any one of claims 1-7, **characterized by** further comprising a second drug, wherein the second drug is selected from one or more of antiemetic agents or analgesic agents.

9. The use according to claim 8, **characterized in that**
the antiemetic agent is selected from tropisetron, ondansetron, granisetron, dolasetron, azasetron, ramosetron, palonosetron, aprepitant, fosaprepitant, rolapitant, casopitant, vetipitant, dimenhydrinate, promethazine, scopolamine, gabapentin, pregabalin, midazolam and ephedrine;
the analgesic agent is selected from morphine, fentanyl, sufentanil, oxycodone, hydromorphone, pethidine, butorphanol, nalbuphine, dezocine, pentazocine, buprenorphine, flurbiprofen axetil, ketorolac, lornoxicam, diclofenac, parecoxib, celecoxib, paracetamol, tramadol, dexmedetomidine, ketamine, esketamine, gabapentin and pregabalin.

10. The use according to any one of claims 5-9, **characterized in that** an effective dose of the compound is selected from 1 ng-30 mg, 0.01 **µg-10 mg,** 0.1 µg-1 **mg,** 1 **µg-1000 µg,** 10 **µg-800 µg,** 10 **µg-600 µg,** 10 **ng-400 µg** and 10 **µg-200 µg,** and effective doses of the hydrate, metabolite, solvate, pharmaceutically acceptable salt or eutectic of the compound, and the composition thereof are converted based on the effective dose of the compound.

11. The use according to claim 10, **characterized in that** an effective dose for intravenous injection is selected from 40 **µg-400 µg** and 60 **µg-180 µg.**
